Europäisches Patentamt

European Patent Office

Office européen des brevets

(11) Publication number: **0 588 255 A1**

## EUROPEAN PATENT APPLICATION

(21) Application number: **93114571.8**

(22) Date of filing: **10.09.93**

(51) Int. Cl.5: **A61K 37/02**, A61K 47/06,
C12N 11/02, C07K 17/02

(30) Priority: **12.09.92 JP 269420/92**

(43) Date of publication of application:
**23.03.94 Bulletin 94/12**

(84) Designated Contracting States:
**AT BE CH DE DK ES FR GB GR IE IT LI LU MC
NL PT SE**

(71) Applicant: **DOTT LIMITED COMPANY
5-3, Fujimigaoka,
Midori-ku
Yokohama, Kanagawa-ken(JP)**

(72) Inventor: **Yanagawa, Akira
5-3, Fujimigaoka,
Midori-ku
Yokohama, Kanagawa-ken(JP)**
Inventor: **Sasaki, Noriyuki
6-6-504, Yakata 2-chome
Shiki-shi, Saitama-ken(JP)**

(74) Representative: **Weisert, Annekäte, Dipl.-Ing.
Dr.-Ing. et al
Patentanwälte
Kraus Weisert & Partner
Thomas-Wimmer-Ring 15
D-80539 München (DE)**

(54) **Physiologically active peptide compositions.**

(57) A physiologically active peptide composition having a physiologically active peptide dispersed homogeneously in and adsorbed homogeneously onto a unique carrier. The physiologically active peptide composition contains a physiologically effective amount of the physiologically active peptide dispersed homogeneously in and adsorbed homogeneously onto the carrier comprising a polyvalent alcohol ester of a polysaccharide selected from alginic acid propylene glycol ester and hyaluronic acid butylene glycol ester. The composition can be nasally administrable in the powdery form or in the form of aqueous solution.

EP 0 588 255 A1

BACKGROUND OF THE INVENTION

1. Field of the Invention

The present invention relates to a physiologically active peptide composition and, more particularly, to a physiologically active peptide composition containing a physiologically active peptide such as a peptide hormone, a physiologically active protein or an enzymatic protein and having high stability in the form of a preparation and improved absorbability into the body, for example, when administered nasally.

2. Description of the Related Art

Physiologically active peptides such as calcitonin and insulin are polymers which are extensively employed in therapeutical treatment for various medical usage due to their specific physiological activity.

These physiologically active peptides, however, can little be absorbed intact from the mucous membrane of the intestine because they are likely to be decomposed with proteases existing in the digestive system or are high in molecular weight and polarity. Hence, they cannot be administered orally and they can be administered only through injection. The injectable administration cannot be said to be preferable because the injection causes pain at the site of injection to patients. In addition, particularly, when the injection should be repeated at constant intervals, such pain is repeated whenever they are injected and it may often become too severe for patients to endure. Hence, strong demand has been made to develop a method for administering the physiologically active peptide via a non-injection route and, more preferably, a method which enables patients to administer it by themselves, which further should be safe, simple in administration and administrable with less frequency.

As one of such methods for administering the physiologically active peptides, an aerosol in the form of a suspension, which uses a fluorinated hydrocarbon as a spouting agent, has been developed for nasally administering, for example, calcitonin. As another means for nasal administration, a spraying agent for nasal administration has been proposed as a nasally administrable liquid preparation, which is a preparation in which calcitonin is formulated with a surface-active agent as an absorption promoter. Furthermore, recently, there have been proposed some nasally administrable powdery preparations having improved absorbability, which are prepared by adsorbing calcitonin onto a polysaccharide such as cellulose. The various techniques for nasal administration, which have recently been actively developed, are said to be in principle superior as methods for administering such physiologically active peptides as unlikely to be administered orally. Since the plexus venosus develops at the nasal lamina propria mucosae of the nasal cavity, the physiologically active peptide, when administered nasally, can be absorbed through the mucous membrane of the nasal cavity into the circulatory system of the body; however, nasally administrable preparations so far proposed cannot be sufficient in terms of absorbability of the physiologically active peptide or local irritability so that they are not commercially available yet.

SUMMARY OF THE INVENTION

The present invention has the object to provide a nasally administrable composition that can nasally administer such a physiologically active peptide as unlikely to be administered orally, with higher bioavailability and less irritability than nasally administrable preparations so far proposed.

As a result of extensive studies and research on such nasally administrable preparations to achieve the object of the present invention, it has been found that there can be administered via a nasal route a composition prepared by homogeneously dispersing the physiologically active peptide, such as calcitonin or insulin, in a particular carrier that has not yet been studied as a carrier for use with a nasally administrable preparation and by homogeneously adsorbing it onto the particular carrier - in other words, that the composition can be applied to the mucous membrane of the nasal cavity - to thereby allow a clinically effective treatment.

In other words, it has been found by the present inventors that the bioavailability can be attained to such an extent as being equal to or higher than that gained by standard injection administration, by the technology that involves homogeneously dispersing the physiologically active peptide such as calcitonin in a unique carrier and adsorbing the physiologically active peptide to the carrier.

It is further found by the present inventors that the unique carrier to be used for the composition according to the present invention can not only act as a carrier for a nasally administrable preparation containing the physiologically active peptide but also offer the effect of promoting the absorption of the physiologically active peptide into the body.

2

The present invention has been completed on the basis of these findings.

In order to achieve the objects of the present invention, a physiologically active peptide composition is provided wherein a physiologically effective amount of a physiologically active peptide is homogeneously dispersed in a carrier comprising a polyvalent alcohol of a polysaccharide selected from an alginic acid propylene glycol ester and a hyaluronic acid butylene glycol ester and the resulting physiologically active peptide is then adsorbed homogeneously onto the carrier.

In other words, the primary object of the present invention is to provide a nasally administrable and physiologically active peptide composition and, more specifically, to provide the composition prepared by homogeneously dispersing the physiologically effective amount of the physiologically active peptide in the alginic acid propylene glycol ester or the hyaluronic acid butylene glycol ester and adsorbing it thereonto homogeneously and, preferably, to provide the composition that is in a powdery form.

The second object of the present invention is to provide a physiologically active peptide composition containing an absorption promoter capable of improving absorbability of the physiologically active peptide into the body and, more specifically, to provide the composition containing, as the absorption promoter, the polyvalent alcohol of the polysaccharide selected from the alginic acid propylene glycol ester and the hyaluronic acid butylene glycol ester, the composition capable of being administered in the form of a nasally administrable aqueous solution or as a rectally administrable preparation.

Other objects, features and advantages of the present invention will become apparent in the course of the description of the preferred embodiments.

## DESCRIPTION OF THE PREFERRED EMBODIMENTS

As described hereinabove, the physiologically active peptide composition according to the present invention is prepared by homogeneously dispersing the physiologically effective amount of the physiologically active peptide in the unique carrier and adsorbing the physiologically active peptide thereonto.

The physiologically active peptide to be used as an active ingredient of the physiologically active peptide composition according to the present invention may include a peptide hormone, a physiologically active protein and an enzymatic protein.

The peptide hormones may include, for example, parathormone (parathyroid hormone), calcitonin, insulin, angiotensin, glucagon, gastrin, secretin, growth hormone, prolactin (luteotropic hormone), gonadotropin (gonodotropic hormone), thyrotropic hormone, adrenocorticotropic hormone), melanocyte stimulating hormone, vasopressin, oxytocin, protirelin, luteinizing hormone releasing hormone, corticotropin, somatropin, thyrotropin (thyroid stimulating hormone), somatostatin (growth hormone inhibiting factor), G-CSF, erythropoietin, superoxide dismutase (SOD), and so on.

The physiologically active proteins may include, for example, interferon, interleukin, and so on.

The enzymatic proteins may include, for example, urokinase, lysozyme, and so on.

In addition, vaccine and so on may also be used as the physiologically active peptide.

It is to be noted herein that the physiologically active peptides to be used for the present invention are not restricted to those described hereinabove and that any nasally administrable physiologically active peptide may be formulated into the physiologically active peptide composition according to the present invention.

Among those physiologically active peptides as described hereinabove, the peptide hormones are preferred. Further, among the peptide hormones, calcitonin, insulin and somatostatin are preferred and calcitonin is particularly preferred.

The calcitonin to be preferably employed for the composition according to the present invention may include, for example, salmon calcitonin, human calcitonin, hog calcitonin, chicken calcitonin, cattle calcitonin, eel calcitonin, and so on. These calcitonins are naturally-occurring ones that are to be extracted from the origin and that are commercially available. It can be noted herein that eel calcitonin is higher in stability than human calcitonin that in turn is higher than salmon calcitonin; however, even the salmon calcitonin relatively low in stability, being homogeneously dispersed into and adsorbed onto the unique carrier to be used for the present invention, can be a physiologically active peptide composition that is high in bioavailability and concentration in blood. Hence, the calcitonins can be most preferred as the physiologically active peptide even in terms of ready availability.

On the other hand, the unique carrier having the physiologically active peptide dispersed thereinto and adsorbed thereonto comprises the polyvalent alcohol of the polysaccharide. The polyvalent alcohol may include, for example, an alkylene glycol such as propylene glycol and butylene glycol and the polysaccharide may include, for example, alginic acid and hyaluronic acid. The alginic acid propylene glycol ester and the hyaluronic acid butylene glycol ester have been used, for example, as an emulsifier or a thickening agent. It should be noted herein, however, that nothing has so far been reviewed about the application of the polyvalent alcohol of the polysaccharide to the carrier for administering a preparation via a nasal route and the present inventors have discovered for the first time that the polyvalent alcohol of the polysaccharide can be employed as a carrier for nasal administration.

The alginic acid propylene glycol ester is commercially available as trade name "KIMILOIDO" and this commercially available ester can be the one that is particularly suitable for the nasal administration through the nasal cavity.

Further, as described hereinabove, it has been found by the present inventors that the unique carrier to be used for the physiologically active peptide composition according to the present invention can not only act as a nasally administrable carrier for nasally administering the physiologically active peptide but also offer the effect for promoting the absorption of the physiologically active peptide into the body.

Hence, the preferred object of the present invention is to provide the physiologically active peptide composition in which the physiologically effective amount of the physiologically active peptide is homogeneously dispersed into and adsorbed thereon and, more specifically, to provide the physiologically active peptide composition administrable through the nasal cavity in the form of an aqueous solution or in the form of a rectally administrable preparation.

Furthermore, the most preferred mode of the composition according to the present invention is the physiologically active peptide composition in the powdery form, which is formulated into the nasally administrable preparation, in which the physiologically effective amount of calcitonin is homogeneously dispersed in and adsorbed onto the alginic acid propylene glycol ester or hyaluronic acid butylene glycol ester.

The physiologically effective amount of the physiologically active peptide to be contained in the physiologically active peptide composition according to the present invention may vary with an individual active substance to be chosen - for example, with a relative activity strength of calcitonin, an objective disease to be treated, a desired number of administration, an effect of necessary therapy, and so on. When the physiologically active peptide composition according to the present invention is to be administered through the nasal cavity, the physiologically effective amount of the physiologically active peptide can be determined on the basis of a comparison of its bioavailability relative to other known preparations containing the same active substance.

For example, when the calcitonin, e.g. salmon calcitonin, is administered intramuscularly for therapy, the dose of the calcitonin ranging from approximately 50 MRC unit (IU) to approximately 100 MRC unit (IU) is applied usually from nearly once per day to nearly three times per week. Hence, when the calcitonin is administered through the nasal route, it is appropriate that the composition be applied at the dose ranging usually from approximately 50 MRC unit (IU) to approximately 400 MRC unit (IU), preferably from approximately 100 MRC unit (IU) to approximately 200 MRC unit (IU), at the rate of from nearly once per day to nearly three times per week.

Hence, for the physiologically active peptide composition according to the present invention, it is convenient to nasally administer the calcitonin once at the dose of from approximately 50 MRC unit (IU) to approximately 400 MRC unit (IU), preferably from approximately 100 MRC unit (IU) to approximately 200 MRC unit (IU).

The physiologically active peptide composition according to the present invention may contain the physiologically active peptide at the rate of from approximately 0.005% to approximately 30%, preferably from approximately 0.01% to approximately 20%, more preferably from approximately 0.1% to approximately 5.0%, with respect to the total weight of the preparation.

On the other hand, when the physiologically active peptide composition according to the present invention may contain the carrier comprising the polyvalent alcohol of the polysaccharide selected from the alginic acid propylene glycol ester or the hyaluronic acid butylene glycol ester at the rate of from 70% to approximately 99.995%, preferably from approximately 80% to approximately 99.99%, more preferably from approximately 95% to approximately 99.9%, with respect to the total weight of the preparation, the physiologically active peptide composition can achieve a high extent of nasal absorption.

Further, when the polyvalent alcohol of the polysaccharide selected from the alginic acid propylene glycol ester and the hyaluronic acid butylene glycol ester is formulated as the absorption promoter with, for example, calcitonin to yield a nasally administrable preparation in the form of aqueous solution, the physiologically active peptide composition according to the present invention in the form of an aqueous solution may be prepared by adjusting an aqueous solution of the physiologically effective amount of the calcitonin to pH in the range of, for example, from pH 4.5 to pH 5.5, preferably nearly pH 5.0, and then adding, for example, gelatin at the rate of approximately 1% or aspartic acid at the rate in the range of from approximately 0.1% to approximately 1%, preferably from approximately 0.2% to 0.4%, as a stabilizing agent, and, thereafter, adding the alginic acid propylene glycol ester or the hyaluronic acid butylene glycol ester as the absorption promoter. When the alginic acid propylene glycol ester or the hyaluronic acid butylene glycol ester is used as the absorption promoter, it may be applied at the rate of from approximately 0.01% to approximately 20%, preferably from approximately 0.1% to approximately 10%, more preferably from approximately 0.5% to approximately 5.0%, with respect to the total weight of the preparation in the form of the aqueous solution.

In addition, the physiologically active peptide composition according to the present invention in the form of a powdery preparation can be formulated by admixing the physiologically active peptide with the particular carrier comprising the polyvalent alcohol of the polysaccharide selected from the alginic acid propylene glycol ester and the hyaluronic acid butylene glycol ester. For the formulation into the powdery preparation of the physiologically active peptide composition according to the present invention, the physiologically active peptide may be admixed with the carrier in a mortar by applying pressure or shear force to the resulting mixture. The polyvalent alcohol of the polysaccharide to be used as the carrier for the preparation for the powdery preparation according to the present invention may have a mean particle size ranging from approximately 20 $\mu$m to approximately 250 $\mu$m, preferably from approximately 30 $\mu$m to approximately 60 $\mu$m. On the other hand, it is preferred that the physiologically active peptide is pulverized to the smallest possible particle size and a mean particle size of the physiologically active peptide may be usually smaller than 20 $\mu$m, preferably smaller than 10 $\mu$m.

From the composition as described hereinabove, the nasally administrable and physiologically active peptide composition according to the present invention can be prepared in such a manner as will be described hereinafter. More specifically, when salmon calcitonin or eel calcitonin is used as the physiologically active peptide, the physiologically effective amount of the calcitonin is admixed with an aqueous solution having from pH 4.5 to pH 5.5 and containing, as the stabilizing agent, gelatin at the rate of, for example, approximately 1% and aspartic acid at the rate of from, for example, approximately 0.1% to 0.5%, preferably approximately 0.38%, and the resulting mixture is then freeze-dried. The resulting powdery mixture is then kneaded at a relative humidity of approximately 55% with two or three portions of the unique carrier comprising the polyvalent alcohol of the polysaccharide selected from the alginic acid propylene glycol ester and the hyaluronic acid butylene glycol ester, thereby yielding fine powder of the objective nasally administrable composition with the physiologically active peptide adsorbed homogeneously on the carrier.

In order for the physiologically active peptide of the physiologically active peptide composition to fail to lose its activity prior to administration into the nasal cavity, the nasally administrable composition may then be filled in capsules of a low-grease type and packaged in an appropriate form, preferably in a closed form, by combining blister packaging with aluminium packaging.

It should be noted that the other physiologically active peptides and the polyvalent alcohol of the polysaccharide can be likewise treated in substantially the same manner as described hereinabove to thereby yield the corresponding composition.

As a result of tests for the preparations conducted in a manner as will be described hereinafter, it is found that a homogenous humidity of the tested preparation is preferably approximately 55%. This will be described later.

The specific effects to be offered by the test examples of the physiologically active peptide compositions according to the present invention will be indicated hereinafter.

Test Example 1:

A nasally administrable composition was prepared by formulating salmon calcitonin as a physiologically active peptide with alginic acid propylene glycol ester as a carrier at the rate of 200 MRC (IU) per 25 mg.

The resulting nasally administrable composition was nasally administered once to six healthy male adults and the blood was collected onto heparin Li salt from each of the tested adults at the rate of 2.5 ml prior to administration and then each at minutes 5, 10, 20, 30, 45, 60, 90, 120, 180 and 300 after administration. The concentration of the salmon calcitonin in each of the gathered blood samples was assayed with a standard RIA assay kit.

The parameters of the pharmacokinetics were computed from the test results and are indicated in Table 1 below.

TABLE 1

| Absorption by Alginic Acid Propylene Glycol Ester | | | | |
|---|---|---|---|---|
| Case Nos. | Tmax (min) | Cmax (pg/ml) | AUC (pg/min/ml) | AUC (pg/hour/ml) |
| 1 | 180 | 56 | 10,627.5 | 177.1 |
| 2 | 20 | 65 | 9,900.0 | 165.0 |
| 3 | 20 | 56 | 7,777.5 | 129.6 |
| 4 | 20 | 45 | 6,117.5 | 102.0 |
| 5 | 20 | 58 | 9,600.0 | 160.0 |
| 6 | 30 | 40 | 6,345.0 | 105.8 |
| Average | 48 | 53 | 8,394.6 | 139.91 |
| S. D. | 65 | 9 | 1,922.1 | 32.0 |

As is apparent from the results indicated in Table 1 above, it is found that the use of the alginic acid propylene glycol ester has demonstrated a high degree of the absorption of the calcitonin into the blood and, consequently, the alginic acid propylene glycol ester is effective for the absorption of the calcitonin through the nasal cavity.

Test Example 2:

A nasally administrable composition in the powdery form was prepared by formulating salmon calcitonin as a physiologically active peptide with hyaluronic acid butylene glycol ester as a carrier at the rate of 200 MRC (IU) per 25 mg.

The resulting nasally administrable composition was nasally administered once at the dose of 25 mg to six healthy male adults and the blood was collected from each of the tested adults prior to administration and then each at minutes 5, 10, 15, 20, 30, 45, 60, 90, 120, 180 and 300 after administration. The concentration of the salmon calcitonin in each of the gathered blood samples was assayed with a standard RIA assay kit.

Table 2 below indicates the change of concentrations of the salmon calcitonin in the blood.

TABLE 2

| | Case Nos. (pg/ml) | | | | | |
|---|---|---|---|---|---|---|
| | 1 | 2 | 3 | 4 | 5 | 6 |
| Before Administration | - | - | - | - | - | - |
| 5 min | - | 16 | 19 | 19 | 18 | - |
| 10 min | 21 | 17 | 17 | 36 | 17 | 22 |
| 15 min | 16 | 16 | 22 | 42 | 27 | 24 |
| 20 min | 23 | 27 | 26 | 29 | 31 | 41 |
| 30 min | 32 | 27 | 43 | 22 | 33 | 35 |
| 45 min | 24 | 22 | 36 | 26 | 20 | 27 |
| 60 min | 18 | 18 | 19 | 18 | 23 | 19 |
| 90 min | - | 18 | 29 | 17 | 16 | - |
| 120 min | 17 | 42 | 22 | - | 18 | - |
| 180 min | - | 17 | 16 | - | - | - |
| 300 min | - | - | - | - | - | - |
| Notes: - Not detected | | | | | | |

As is apparent from the results indicated in Table 2 above, it is found that the composition in the powdery form has demonstrated a high degree of the absorption of the calcitonin into the blood and, as a result, the hyaluronic acid butylene glycol ester is effective for the nasal absorption of the calcitonin.

Test Example 3:

The nasally administrable and physiologically active peptide composition in the form of aqueous solution was prepared from salmon calcitonin as the physiologically active peptide and the hyaluronic acid butylene glycol ester as the absorption promoter so as to contain the salmon calcitonin at the rate of 400 MRC (IU) per 10 ml.

The aqueous solution of the physiologically active peptide composition was prepared by adjusting 100 ml of purified water with hydrochloric acid to pH 5.0 and then admixing the resulting mixture with predetermined amounts of salmon calcitonin, benzalkonium chloride (0.01%), gelatin (1%), aspartic acid (0.38%), and hyaluronic acid butylene glycol ester (2.0%).

The resulting nasally administrable composition was nasally administered once at the dose of 10 ml to six healthy male adults and the blood was collected from each of the tested adults prior to administration and then each at minutes 5, 10, 15, 20, 30, 45, 60, 90, 120, 180 and 300 after administration. The concentration of the salmon calcitonin in each of the gathered blood samples was assayed with a standard RIA assay kit.

Table 3 below indicates the change of concentrations of the salmon calcitonin in the blood.

TABLE 3

| | Case Nos. (pg/ml) | | | | | |
|---|---|---|---|---|---|---|
| | 1 | 2 | 3 | 4 | 5 | 6 |
| Before Administration | - | - | - | - | - | - |
| 5 min | 16 | 24 | 17 | 16 | 42 | 18 |
| 10 min | 16 | 18 | 19 | 19 | 22 | 21 |
| 15 min | 19 | 23 | 17 | 18 | 17 | 23 |
| 20 min | 16 | 33 | 23 | 16 | 16 | 17 |
| 30 min | 20 | 49 | 27 | 22 | 17 | 16 |
| 45 min | 16 | 17 | 24 | 23 | 26 | 17 |
| 60 min | 16 | 40 | 25 | 16 | 18 | 16 |
| 90 min | 16 | 30 | 30 | 18 | 18 | 16 |
| 120 min | 16 | 40 | 27 | 20 | 17 | 19 |
| 180 min | 17 | 32 | 24 | 22 | 50 | 22 |
| 300 min | 16 | 29 | 22 | 16 | 16 | 22 |
| Notes: - Not detected | | | | | | |

As is apparent from the results indicated in Table 3 above, it is found that the composition in the form of the aqueous solution has demonstrated a high degree of the effect of enhancing the absorption of the calcitonin into the blood and, consequently, the hyaluronic acid butylene glycol ester is effective for the absorption of the calci-tonin through the nasal cavity.

The physiologically active peptide compositions according to the present invention are then tested for their effects as preparations.

Test Example 4:

The carrier (200 mg) having a mean particle size of approximately 40 μm, as indicated in Table 4 below, was admixed with salmon calcitonin (5,500 MRC (IU)/mg) and pulverized at 4° C in an agate mortar.

From the powdery mixture, calcitonin unadsorbed on the carrier particles was separated and removed and the amount of the salmon calcitonin adsorbed thereon was measured. The results are shown in Table 4 below.

TABLE 4

| Kind of Carriers | Amount of Salmon Calcitonin | |
|---|---|---|
| | ng/mg | MRC/mg |
| Sodium alginate | 229 | 1.26 |
| Propylene glycol alginate | 2,250 | 12.4 |
| Pectin | 105 | 0.58 |
| Chitosan | 2,680 | 14.7 |
| Hyaluronic acid | 1,130 | 6.22 |

Table 4 above shows that the alginic acid propylene glycol ester used as the carrier can demonstrate a high degree of the effect of adsorbing the calcitonin thereon.

EP 0 588 255 A1

Test Example 5:

The composition prepared from the alginic acid propylene glycol ester as the carrier was tested for stability as a preparation.

The composition was prepared by admixing given amounts of salmon calcitonin with an aqueous solution having pH 5.0 and containing aspartic acid at the rate of 0.38% and lyophilizing the resulting mixture. The lyophilized mixture was then admixed homogeneously with 1 gram of alginic acid propylene glycol ester as a carrier at temperature of 5° C, 25° C or 40° C and humidity of 25%, 55% or 65%.

The alginic acid propylene glycol ester used as the carrier had a mean particle size in the range of from 30 μm to 60 μm.

The resulting composition was filled in a glass bottle and allowed to stand for 6 months. The amount of the salmon calcitonin left active was measured in one month, three months and six months after the start of storage. The amounts of the salmon calcitonin left active are measured and represented in percentage (%). The results are shown in Table 5 below.

TABLE 5

| Conditions | | Storage Period | | |
|---|---|---|---|---|
| Temperature | Humidity | 1 month | 3 months | 6 months |
| 40° C | 25% | 96.5 | 92.0 | 86.0 |
| | 55% | 86.8 | 79.2 | 72.4 |
| | 65% | 85.5 | 81.3 | 77.7 |
| 25° C | 25% | 99.8 | 98.5 | 97.3 |
| | 55% | 97.6 | 95.1 | 93.2 |
| | 65% | 95.1 | 93.4 | 91.2 |
| 5° C | 25% | 101.3 | 99.5 | 99.2 |
| | 55% | 97.3 | 97.3 | 96.7 |
| | 65% | 98.7 | 97.8 | 97.1 |

As is apparent from the results as indicated in Table 5 above, it has been found that the compositions according to the present invention can offer higher stability when the particle sizes of the carriers are smaller to some extent and when the humidity are usually in the range of 40% to 60% although it may depend upon the temperature.

The physiologically active peptide compositions according to the present invention may be formulated into various preparations in a manner will be described hereinafter.

Powdery Preparation 1:

Salmon calcitonin (3 mg/3,000-3,500 MRC) was dissolved in an aqueous solution having from pH 4.5 to pH 5.5 and containing gelatin at the rate of 1% and aspartic acid as a stabilizing agent at the rate of 0.38%, and the resulting mixture solution was lyophilized. The lyophilized mixture was then admixed in an agate mortar with 200 mg of alginic acid propylene glycol ester for 10 minutes and an additional amount of 300 mg of alginic acid propylene glycol ester was further added. The resulting mixture was stirred at humidity of 55% for 20 minutes. Further, 497 mg of alginic acid propylene glycol ester was added and the mixture was admixed for 30 minutes at humidity of 55%, thereby yielding 1,00 mg of a powdery composition.

Powdery Preparation 2:

The powdery preparation was prepared in substantially the same manner as the procedure for the production of the powdery preparation 1 above, except for using eel calcitonin in place of salmon calcitonin.

9

Aqueous Solution:

An aqueous solution was prepared by adjusting 100 ml of purified water with hydrochloric acid to pH 5.0, and there were added 4 mg of salmon calcitonin (4,000 MRC), benzalkonium chloride at the rate of 0.01%, gelatin at the rate of 1%, aspartic acid at the rate of 0.38%, and hyaluronic acid butylene glycol ester at the rate of 2%. The resulting mixture was admixed homogeneously to thereby yield the composition in the form of aqueous solution. Rectally Administrable Preparation (Suppository):

```
Formulation 1:

    Salmon calcitonin            200 MRC

    Purified water                   Given amount

       (pH 4.0 with 0.1N HCl; containing

        aspartic acid           0.38%; and

        gelatin                 1%)

    Propylene glycol alginate        10 mg

    Hydrophilic base                 Given amount

         Total weight:           2-4 grams
```

As the hydrophilic base, there was used glycerinated gelatin and the rectally administrable preparation in the form of a suppository was prepared in conventional manner.

The concentration of the salmon calcitonin was measured when the resulting suppository was used. As a result, it was found that the concentration of the salmon calcitonin reached its maximal level in 10 minutes or 20 minutes after administration. Hence, the alginic acid propylene glycol ester used for the composition according to the present invention has demonstrated a high degree of the effect for promoting the absorption of the salmon calcitonin.

Further, it can be noted that the rectally administrable preparations can be prepared in substantially the same manner as with other hydrophilic bases such as polyethylene glycol (Macrogol), gelatin capsules, and emulsifiable bases. These preparations have also exhibited substantially the same effect as the rectally administrable preparation prepared using the glycerinated gelatin.

Formulation 2:

| | |
|---|---|
| Salmon calcitonin | 200 MRC |
| Purified water | Given amount |
| (pH 4.0 with 0.1N HCl; containing | |
| aspartic acid | 0.38%; and |
| gelatin | 1%) |
| Propylene glycol alginate | 10 mg - 25 mg |
| Surface-active agent | Given amount |
| Lipophilic base | Given amount |
| Total weight: | 2-4 grams |

When the lipophilic base is employed, it is preferred to add a compatible surface-active agent for setting an HLB value to approximately 4-6. In the formulation as specified above, there was used a mixture of mono-, di- and tri-glycerides of middle-chained fatty acid (e.g. Witepsol), as the lipophilic base, and dipotassium glycyrrhizinate, as the surface-active agent. This rectally administrable composition (suppository) was prepared by a conventional method for preparing suppositories.

The rectally administrable preparations can be prepared in substantially the same manner by using other lipophilic bases such as Estarium A, B, C, D, E, TV, Novata A, AB, BC, Suppocire A, B, C, AM, BM, and natural cacao oil. Likewise, they can be prepared in substantially the same manner by using other surface-active agents.

As described hereinabove, the physiologically active peptide compositions according to the present invention allows the physiologically active peptides, which are unlikely or difficult to be orally administered, to be administered through a nasal route with high absorbability and without irritability.

In particular, when the composition in the powdery form having the physiologically active peptide such as calcitonin or insulin dispersed in the particular carrier such as alginic acid propylene glycol ester or hyaluronic acid butylene glycol ester is administered through the nasal route, that is, when it is applied to the mucous membrane of the nasal cavity, the physiologically active peptide can be absorbed into the body with high absorbability and it can work with high clinical effects. Further, the alginic acid propylene glycol ester and the hyaluronic acid butylene glycol ester to be used for the present invention can offer the effects of enhancing the absorption of the physiologically active peptides. Hence, the composition in the form of aqueous solution containing the particular carrier can increase the absorbability of the physiologically active peptide into the body when rectally administered through the nasal route or as a suppository.

The physiologically active peptide compositions according to the present invention can provide a great extent of the clinical and curable effects.

**Claims**

1. A physiologically active peptide composition comprising a physiologically active peptide and a carrier, wherein a physiologically effective amount of said physiologically active peptide is dispersed homogeneously in and adsorbed homogeneously onto said carrier comprising a polyvalent alcohol of a polysaccharide selected from an alginic acid propylene glycol ester and a hyaluronic acid butylene glycol ester.

2. A physiologically active peptide composition as claimed in claim 1, wherein aspartic acid is further contained as a stabilizing agent.

3. A physiologically active peptide composition as claimed in claim 1, wherein said physiologically active peptide comprises a peptide hormone, a physiologically active protein or an enzymatic protein.

4. A physiologically active peptide composition as claimed in claim 3, wherein said peptide hormone is calcitonin.

5. A physiologically active peptide composition as claimed in claim 1, wherein said physiologically active peptide is calcitonin and said carrier is alginic acid propylene glycol ester and said calcitonin is dispersed homogeneously in and adsorbed homogeneously onto said alginic acid propylene glycol ester.

6. A physiologically active peptide composition as claimed in claim 1, wherein said physiologically active peptide is calcitonin and said carrier is hyaluronic acid butylene glycol ester and said calcitonin is dispersed homogeneously in and adsorbed homogeneously onto said hyaluronic acid butylene glycol ester.

7. A physiologically active peptide composition as claimed in any one of claims 1 to 6, wherein said composition is nasally administrable in a powdery form.

8. A physiologically active peptide composition as claimed in claim 7, wherein said calcitonin is dispersed homogeneously in and adsorbed homogeneously onto said alginic acid propylene glycol ester.

9. A physiologically active peptide composition as claimed in claim 7, wherein said calcitonin is dispersed homogeneously in and adsorbed homogeneously onto said hyaluronic acid butylene glycol ester.

10. A physiologically active peptide composition wherein a physiologically effective amount of a physiologically active peptide is formulated with an absorption promoter selected at least one from alginic acid propylene glycol ester and hyaluronic acid butylene glycol ester.

11. A physiologically active peptide composition as claimed in claim 10, wherein said stabilizing agent is aspartic acid.

12. A physiologically active peptide composition as claimed in claim 10, wherein said composition is in a form of aqueous solution.

13. A physiologically active peptide composition as claimed in claim 12, wherein said composition is nasally administrable in the form of aqueous solution.

14. A physiologically active peptide composition as claimed in claim 10, wherein said composition is rectally administrable.

| DOCUMENTS CONSIDERED TO BE RELEVANT | | | EP 93114571.8 | |
|---|---|---|---|---|
| **Category** | **Citation of document with indication, where appropriate, of relevant passages** | **Relevant to claim** | **CLASSIFICATION OF THE APPLICATION (Int. Cl.5)** | |
| A | DE - A - 3 835 099 (DEBIOPHARM)   * Claims 1,7,8 *   -- | 1,3,4 | A 61 K 37/02 A 61 K 47/06 C 12 N 11/02 C 07 K 17/02 | |
| A | CH - A - 621 146 (BOEHRINGER MANNHEIM)   * Claims 1,7 *   ---- | 1 | | |
| | | | **TECHNICAL FIELDS SEARCHED (Int. Cl.5)** | |
| | | | A 61 K C 12 N C 07 K | |
| | The present search report has been drawn up for all claims | | | |

| Place of search | Date of completion of the search | Examiner |
|---|---|---|
| VIENNA | 09-12-1993 | WOLF |

EPO FORM 1503 03.82 (P0401)